(19) Europäisches Patentamt
European Patent Office
Office européen des brevets

(11) Publication number: **0 029 310**
**B1**

(12) **EUROPEAN PATENT SPECIFICATION**

(45) Date of publication of patent specification: **03.08.83**

(51) Int. Cl.³: **C 07 C 103/52, A 61 K 37/02**

(21) Application number: **80303826.4**

(22) Date of filing: **28.10.80**

(54) **Cyclic hexapeptide somatostatin analogs, process for their preparation and pharmaceutical compositions containing them.**

(30) Priority: **31.10.79 US 89827**
**31.10.79 US 89828**

(43) Date of publication of application:
**27.05.81 Bulletin 81/21**

(45) Publication of the grant of the patent:
**03.08.83 Bulletin 83/31**

(84) Designated Contracting States:
**AT BE CH DE FR GB IT LI LU NL SE**

(56) References cited:
**EP - A - 0 001 295**
**DE - A - 2 734 628**

**The file contains technical information submitted after the application was filed and not included in this specification**

(73) Proprietor: **MERCK & CO. INC.**
**126, East Lincoln Avenue P.O. Box 2000**
**Rahway New Jersey 07065 (US)**

(72) Inventor: **Freidinger, Roger M.**
**2185 Rebecca Drive**
**Hatfield Pennsylvania 19440 (US)**
Inventor: **Veber, Daniel F.**
**290 Batleson Road**
**Ambler Pennsylvania 19002 (US)**

(74) Representative: **Crampton, Keith John Allen et al,**
**D YOUNG & CO 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

**EP 0 029 310 B1**

## Cyclic hexapeptide somatostatin analogs

Somatostatin is a tetradecapeptide incorporating a cyclic dodecapeptide and has the structural formula:

Ala–Gly–Cys–Lys–Asn–Phe–Phe–Trp–Lys–Thr–Phe–Thr–Ser–Cys–OH
1 2 3 4 5 6 7 8 9 10 11 12 13 14

It has the properties of inhibiting the release of growth hormone, inhibiting the release of insulin and glucagon and reducing gastric secretions. Somatostatin itself has a short duration of action because it is inactivated, *inter alia,* by aminopeptidases and carboxypeptidases present *in vivo*. This problem of the short duration of action has been partially solved in the prior art by preparing derivatives of somatostatin which have low solubility, thus attaining a slow release on subcutaneous injection. Once dissolved, however, the derivatives are no more stable to inactivation by aminopeptidases and carboxypeptidases than somatostatin itself.

European Patent Specification EU—A2—0001295 discloses *inter alia* the Somatostatin analogues of the formula:

Phe-Phe-Trp*-Lys-Thr-Phe

in which Trp* is D-Trp or L-Trp.

The present invention is concerned with cyclic hexapeptides that are derivatives of somatostatin in which, *inter alia,* seven of the ring amino acids are replaced by a secondary amino acid, and both of the exocyclic amino acids are removed. Further substitution and reaction of the remaining amino acids is also possible. Cyclic hexapeptides of the present invention inhibit the release of glucagon, growth hormones and insulin, and inhibit the release of gastric acid secretions. Specifically such compounds may preferentially inhibit the release of growth hormones without effecting the level of gastric secretions, insulin and glucagon, or the compounds may inhibit the release of gastric acid secretions. Thus, the compounds of the present invention may have a more selective bioligical activity than somatostatin. The cyclic hexapeptide structure of the compounds of the present invention also has a longer duration of activity than somatostatin. As such, the cyclic hexapeptides of the present invention are useful for the treatment of acromegaly, diabetes, diabetic retinopathy and peptic ulcers.

The compounds of the present invention have the structural formula:

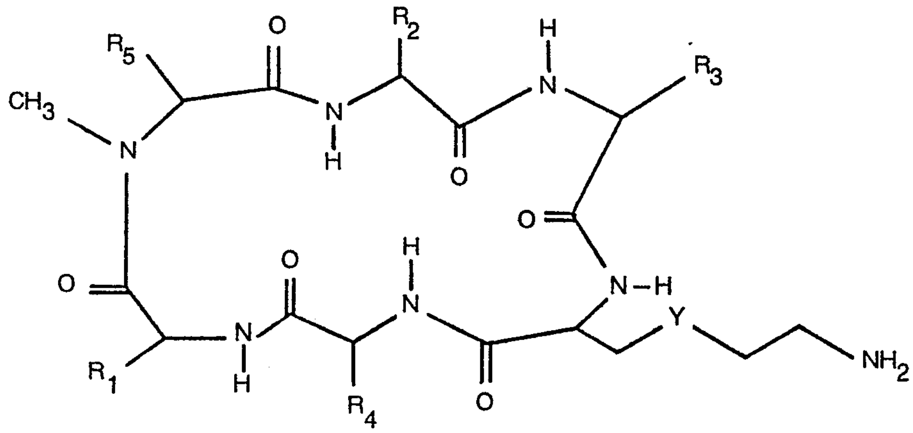

I

or

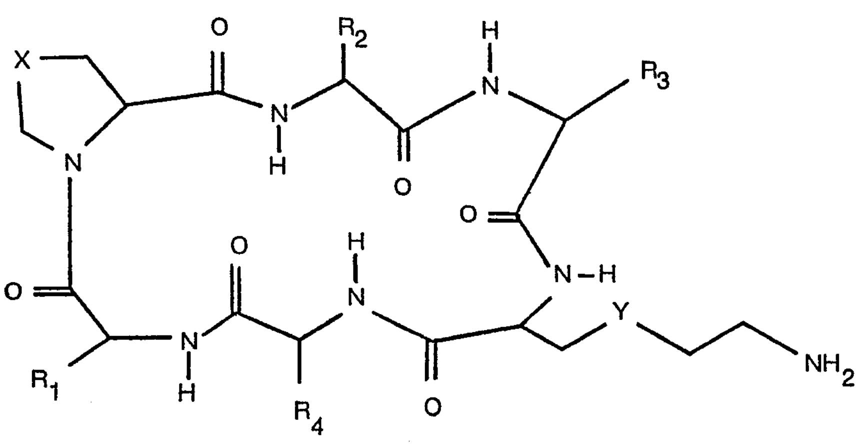

II

in which X is $(CH_2)_n$, where $n$ is 0, 1 or 2, or X is sulfur;

Y is $(CH_2)_m$, where $m$ is 0, 1 or 2, or Y is sulfur such that the sulfur may be in any position along the chain;

each of $R_1$ and $R_2$, independently of the other, is lower alkyl, benzyl, substituted benzyl where the substituent is one or two of lower alkyl, halogen, hydroxy, amino, nitro and lower alkoxy; or lower alkyl substituted with a 5-membered or 6-membered heterocyclic ring;

$R_3$ is 3-indolymethyl or substituted 3-indolylmethyl in which the substituent is lower alkyl, lower alkoxy or halogen;

$R_4$ is lower alkyl, lower hydroxyalkyl, benzyl, carboxy-(lower alkyl), lower aminoalkyl or substituted benzyl in which the substituent is lower alkyl, lower alkoxy, hydroxy, halogen, amino or nitro; and

$R_5$ is lower alkyl, benzyl, or substituted benzyl in which the substituent is lower alkyl, lower alkoxy, hydroxy, halogen, amino or nitro.

The term "lower alkyl" when used in the present application means $C_{1-5}$ alkyl, either straight or branched chain, e.g. methyl, ethyl, propyl, *iso*-propyl, butyl, *sec*-butyl or pentyl.

The term "lower alkoxy" means alkoxy of from 1—5 carbon atoms, in either a straight or branched chain. Examples of such alkoxy groups are methoxy, ethoxy, propoxy, isopropoxy, butoxy, *tert*-butoxy and pentoxy.

The term "halogen" or "halo" means fluorine, chlorine, bromine or iodine.

The term "5- or 6-membered heterocyclic ring" is intended to include those 5- and 6-membered heterocycles with one or two heteroatoms selected from oxygen, nitrogen and sulfur. Examples of such heterocycles are imidazole, furan, thiazole, pyrazole and pyridine.

In the compounds of the present invention there are several asymmetric centers, which will lead to the existence of optical isomers for such compounds. In the present compounds, for each of the asymmetric centers of the various amino acids that make up the cyclic hexapeptides, both the *D* and *L* configurations are intended to be encompassed.

It will be appreciated (a) that when $R_1$ and $R_2$ are benzyl, $R_3$ is indolylmethyl, Y is methylene, and $R_4$ is 1-hydroxyethyl, the 7,8,9,10 and 11 amino acids of somatostatin (-Phe-Trp-Lys-Thr-Phe-) are represented, and (b) that the secondary amino acid, represented by N-methyl alanine when $R_5$ is methyl, in structure I, and by proline when X is methylene in structure II, has taken the place of the remainder of the somatostatin amino acids. Thus, using the above definitions of the substituent groups, the following representative cyclic hexapeptide analog of somatostatin is formed in structure I;

```
N-Me-Ala-Phe-Trp
        |       |
        Phe-Thr-Lys
```

The preferred cyclic hexapeptides of this invention are those of formula I in which Y is methylene;
$R_1$ and $R_2$ are as defined above;
$R_3$ is 3-indolylmethyl or substituted indolylmethyl where the substituent is methoxy or fluoro;
$R_4$ is methyl, ethyl, hydroxymethyl or hydroxyethyl; and
$R_5$ is methyl.

Particularly preferred compounds are those in which when Y is methylene;
$R_1$ and $R_2$ are as defined above;
$R_3$ is 3-indolylmethyl;
$R_4$ is hydroxyethyl, and
$R_5$ is methyl.

The preferred $R_1$ and $R_2$ groups are lower alkyl, benzyl or substituted benzyl where the substituent is lower alkyl, halogen, hydroxy, amino, nitro or alkoxy.

Included within these preferred compounds are:

Cyclo(N-Me-Ala-Tyr-D-Trp- Lys-Thr-Phe)
Cyclo-(N-Me-Ala-Phe-D-Trp-Lys-Thr-Phe)
Cyclo-(N-Me-Ala-Phe-L-Trp-Lys-Thr-Phe)
Cyclo-(N-Me-Ala-Phe-D-Trp-Lys-Thr-p-Cl-Phe)
Cyclo-(N-Me-Ala-Phe-D-5-F-Trp-Lys-Thr-Phe)
Cyclo(N-Me-Ala-Phe-L-5-F-Trp-Lys-Thr-Phe)
Cyclo-(N-Me-Ala-Phe-D-Trp-Lys-Ser-Phe)

Using the above definitions of the substituent groups, the following representative cyclic hexapeptide analog of somatostatin is formed in structure II:

```
Pro-Phe-Trp
 |       |
Phe-Thr-Lys
```

The preferred embodiments of the cyclic hexapeptides of this invention are realized in the foregoing structural formula II wherein X and Y are $(CH_2)_n$ and n is 1;

$R_1$ and $R_2$ are as defined above;

$R_3$ is 3-indolylmethyl or substituted indolylmethyl wherein the substituent is methoxy or fluoro; and

$R_4$ is methyl, ethyl, hydroxy methyl or hydroxy ethyl.

Further preferred embodiments are realized when X and Y are methylene;

$R_1$ and $R_2$ are as defined above;

$R_3$ is 3-indolylmethyl; and

$R_4$ is hydroxy ethyl.

The preferred $R_1$ and $R_2$ groups are loweralkyl, benzyl or substituted benzyl where the substituent is loweralkyl, halogen, hydroxy, amino, nitro or alkoxy.

Included within these preferred compounds are:

Cyclo-(Pro-Tyr-D-Trp-Lys-Thr-Phe)
Cyclo(Pro-Phe-D-Trp-Lys-Thr-Phe)
Cyclo(Pro-Phe-L-Trp-Lys-Thr-Phe)
Cyclo-(Pro-Phe-D-Trp-Lys-Thr-p-Cl-Phe)
Cyclo(Pro-Phe-D-5-F-Trp-Lys-Thr-Phe)
Cyclo-(Pro-Phe-L-F-Trp-Lys-Thr-Phe)
Cyclo-(Pro-Phe-D-Trp-Lys-Ser-Phe)

In the instant application several abbreviated designations are used for the amino acid components, certain preferred protecting groups, reagents and solvents. The meaning of such abbreviated designations are given in Table I.

TABLE I

| *Abbreviated Designation* | *Amino Acid* |
|---|---|
| Lys | L-lysine |
| Phe | L-phenylalanine |
| Trp | L-tryptophan |
| D-Trp | D-tryptophan |
| Thr | L-threonine |
| Aha | 7-aminoheptanoic acid |
| Tyr | L-tyrosine |
| Val | L-valine |
| Abu | L-$\alpha$-aminobutyric acid |
| Ser | L-serine |
| Asn | L-asparagine |
| Pro | L-proline |
| Asu | D- or L-aminosuberic acid |
| Cys | L-cysteine |

| *Abreviated Designation* | *Protecting Groups* |
|---|---|
| INOC | isonicotinyloxycarbonyl |
| BOC | tert-butyloxycarbonyl |
| OMe | methyl ester |
| Bu | tert-butyl |
| CBz | benzyloxycarbonyl |
| Bzl | benzyl |
| 2-Cl-CBZ | 2-chlorobenzyloxycarbonyl |
| Acm | acetamidomethyl |
| Me | methyl |

| *Abbreviated Designation* | *Activating Groups* |
|---|---|
| ONp | p-nitrophenyl ester |
| HSE | N-hydroxysuccinimide ester |
| HBT | 1-hydroxybenzotriazole |

| *Abbreviated Designation* | *Condensing Agents* |
|---|---|
| DCCI | dicyclohexylcarbodiimide |

| *Abbreviated Designation* | *Reagents* |
|---|---|
| TFA | trifluoroacetic acid |
| TEA | triethylamine |
| DIPEA | diisopropylethylamine |

| *Abbreviated Designation* | *Solvents* |
|---|---|
| EPAW | ethyl acetate-pyridine-acetic acid-water |
| BAW | butanol-acetic acid-water |
| CMW | chloroform-methanol-water |
| DMF | dimethylformamide |
| THF | tetrahydrofuran |

In accordance with the present invention, the novel cyclic hexapeptide somatostatin analogs are prepared by cyclizing corresponding linear peptides. The linear peptides are prepared by using the solid phase sequential synthesis technique. Accordingly, the process for preparing the cyclic hexapeptide somatostatin analogs of the present invention comprises a) preparing a corresponding blocked linear peptide attached to a solid phase resin; b) selectively deblocking the N-terminal amine group; c) removing the linear peptide from the resin; d) treating the linear peptide with a cyclizing agent to obtain the cyclic hexapeptide through the formation of an amide bond; e) removing any side chain blocking groups.

When the linear peptide is prepared on the resin, it is generally not critical which amino acid is selected to be at the C-terminal position provided only that the sequence of amino acids in the linear peptide corresponds to that in the desired somatostatin analog. Once a linear peptide has been cyclized one can no longer determine which amino acid was at the C-terminus of the linear peptide.

While generally the selection of the first amino acid to start the chain is not critical, since the linear peptide will be cyclized, there may be other factors which may prefer one starting amino acid over another. For example D-Trp can react with t-butyl carbonium ions which are formed when BOC groups are removed. Thus, selection of a reaction sequence which places D-Trp at the N-terminal end of the linear peptide will cause D-Trp to be added last, and thys it will have the least exposure to t-butyl carbonium ions. This type of selection may not always be possible, such as where there are two indole-containing moieties in the peptide. However, such reaction sensitivities should be considered when planning a peptide reaction sequence.

The synthesis of the linear peptides by the solid phase technique is conducted in a stepwise manner on chloromethylated resin. The resin is composed of fine beads (20—70 microns in diameter) of a synthetic resin prepared by copolymerization of styrene with 1—2 percent divinylbenzene. The benzene rings in the resin are chloromethylated in a Friedel-Crafts reaction with chloromethyl methyl ether and stannic chloride. The Friedel-Crafts reaction is continued until the resin contains 0.5 to 5 mmoles of chlorine per gram of resin.

The amino acid selected to be the C-terminal amino acid of the linear peptide is converted to its amino protected derivative. The carboxyl group of the selected C-terminal amino acid is bound covalently to the insoluble polymeric resin support, as for example, as the carboxylic ester of the resin-bonded benzyl chloride present in chloromethyl-substituted polystyrene-divinylbenzene resin. After the amino protecting group is removed, the amino protected derivative of the next amino acid in the sequence is added along with a coupling agent, such as dicyclohexylcarbodiimide. The amino acid reactant may be employed in the form of a carboxyl-activated amino acid such as the ONp ester, an amino acid azide, and the like. Deprotection and addition of successive amino acids is performed until the desired linear peptide is formed.

The selection of protecting groups is, in part, dictated by particular coupling conditions, in part by the amino acid and peptide components involved in the reaction.

Amino-protecting groups ordinarly employed include those which are well known in the art, for example, urethane protecting substituents such as benzyloxycarbonyl (carbobenzoxy), p-methoxy-carbobenzoxy, p-nitrocarbobenzoxy, t-butyloxycarbonyl, and the like. It is preferred to utilize t-butyloxycarbonyl (BOC) for protecting the $\alpha$-amino group in the amino acids undergoing reaction at the carboxyl end of said amino acid. The BOC protecting group is readily removed following such coupling reaction and prior to the subsequent step by the relatively mild action of acids (i.e. trifluoro acetic acid, or hydrogen chloride in ethyl acetate).

The OH group of Thr and Ser can be protected by the Bzl group and the $\varepsilon$-amino group of Lys can be protected by the INOC group or the 2-chlorobenzyloxycarbonyl (2-Cl-CBZ) group. In the case of Lys, it is preferred to protect the $\varepsilon$-amino group with 2-Cl-CBZ group as this group is removed simultaneously with the Bzl groups by treatment with HF after the linear peptide has been cyclized. The INOC group is not removed by HF and requires an additional treatment with Zn. Neither group is affected by TFA, used for removing BOC protecting groups. After the linear peptide is cyclized, the protective groups, such as 2-Cl-CBZ and Bzl, are removed by treatment with HF.

After the linear peptide has been formed on the solid phase resin, it may be removed from the resin by a variety of methods which are well known in the art. For example the peptide may be cleaved from the resin with hydrazine and thus directly form the peptide hydrazide which may be subsequently cyclized via the azide to the desired cyclic peptide. The hydrazide is converted to the corresponding azide by reaction with a reagent which furnishes nitrous acid *in situ*. Suitable reagents for this purpose include a lower alkyl nitrite (e.g. t-butyl nitrite, isoamyl nitrite) or an alkali metal nitrite salt (e.g., sodium nitrite, potassium nitrite) in the presence of a strong acid such as hydrochloric, phosphoric, etc. This reaction is carried out in the presence of either water and/or a non-aqueous solvent such as dimethylformamide, tetrahydrofuran, dioxane, chloroform, methylene chloride, etc., at a temperature between about −40°C and +20°C. Alternatively, the peptide may be removed from the resin by treatment with a lower alcohol such as methanol in the presence of an organic base such as triethylamine, thus resulting in the formation of the corresponding lower alcohol ester of the linear peptide. The resulting ester may be converted to the hydrazide which may then be cyclized, via the azide, to the desired cyclic

peptide. The preferred method for cleaving the peptide from the resin in the present invention is the use of hydrazine.

As reference Table II will show, one preferred overall procedure for preparing the desired cyclic peptides of the present invention involves the stepwise synthesis of the linear peptide on a solid phase resin. More specifically, in the process for preparing:

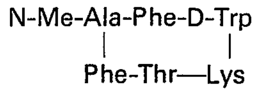 or 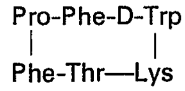

the carboxyl end of the N-blocked amino acid phenylalanine is bound covalently to an insoluble polymeric resin support as the carboxylic acid ester of the resin-bonded benzyl chloride. The amino group of Phe is protected by the BOC group. After the attachment of the Phe is completed on the resin, the protecting group BOC is removed by treatment with TFA in $CH_2Cl_2$. The subsequent amino acids are attached, in the form of BOC-amino acid, using DCCI as the condensing agent or an active ester such as ONp. After the desired linear peptide has been prepared, the N-terminal amino group is selectively deblocked and the peptide is removed from the resin by treatment with hydrazine. The resulting linear peptide hydrazide with the N-terminal amino group deblocked having the amino acid sequence:

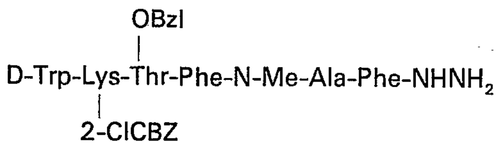 or 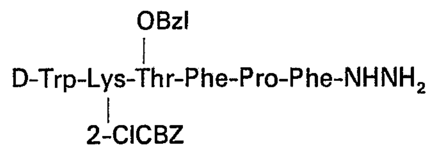

is treated with isoamyl nitrite in acid pH to form the corresponding azide. The azide solution is diluted with solvent and neutralized with an organic base. The linear peptide cyclizes to form:

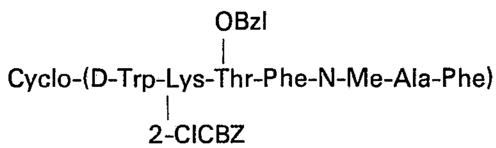

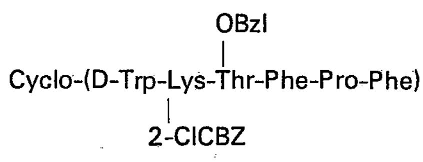

During the cyclization the "pH" is checked and maintained at neutral by the addition of organic base. The "pH" in organic solvent is determined by the application of an aliquot of the solution to moistened narrow range pH paper.

After the linear peptide is cyclized, the protective groups, 2-Cl-CBZ and OBzl, are removed by treatment with HF in the presence of anisole. The crude cyclic peptide obtained is purified chromatographically, preferably with column chromatography on silica gel. The elution solvent is generally an organic solvent or mixtures thereof which is selected by analyzing aliquots of the material using thin layer chromatography.

TABLE II

The reaction scheme for the preparation of two of the cyclic hexapeptides of this invention is outlined in the following series of reactions:

Reaction scheme for preparing:

N-Me-Ala-Phe-D-Trp
|          |
Phe-Thr—Lys

Cl-$CH_2$Ø-resin

BOC-Phe

BOC—Phe-O—$CH_2$Ø-resin

Solid Phase Method

1) BOC—N—Me-Ala

2) BOC-Phe

3) BOC—(OBZl)Thr

4) BOC-(ε-2-Cl-CBZ)Lys

5) BOC—D-Trp

BOC—D-Trp(ε-2-Cl-CBZ)Lys-(OBZl)Thr-Phe-N-Me-Ala-Phe-$OCH_2$Ø-resin

TFA

D-Trp-(ε-2-Cl-CBZ)Lys-(OBZl)Thr-Phe-N-Me-Ala-Phe-O—$CH_2$Ø-resin

$NH_2NH_2$

D-Trp-(ε-2-Cl-CBZ)Lys-(OBZl)Thr-Phe-N-Me-Ala-Phe-$NHNH_2$

isoamyl nitrite, $H^+$ DMF

D-Trp-(ε-2-Cl-CBZ)Lys-(OBZl)Thr-Phe-N-Me-Ala-Phe-$N_3$

DMF/triethylamine

Cyclo(D-Trp-(ε-2-Cl-CBZ)Lys-(OBZl)Thr-Phe-N-Me-Ala-Phe)

HF/Anisole

Cyclo-(D-Trp-Lys-Thr-Phe-N-Me-Ala-Phe)

Reaction scheme for preparing:

Pro-Phe-D-Trp
|          |
Phe-Thr—Lys

Cl-$CH_2$Ø-resin

BOC-Phe

BOC-Phe-O—$CH_2$Ø-resin

Solid Phase Method

1) BOC-Pro

2) BOC-Phe

3) BOC—(OBZl)Thr

4) BOC-(ε-2-Cl-CBZ)Lys

5) BOC—D-Trp

TABLE II (continued)

↓

BOC—D-Trp(ε-2-Cl-CBZ)Lys-(OBZl)Thr-Phe-Pro-Phe-OCH$_2$Ø-resin

↓ TFA

D-Trp-(ε-2-Cl-CBZ)Lys-(OBZl)Thr-Phe-Pro-Phe-O—CH$_2$Ø-resin

↓ $NH_2NH_2$

D-Trp-(ε-2-Cl-CBZ)Lys-(OBZl)Thr-Phe-Pro-Phe-NHNH$_2$

↓ isoamylnitrite, $H^+$, DMF

D-Trp-(ε-2-Cl-CBZ)Lys-(OBZl)Thr-Phe-Pro-Phe-N$_3$

↓ DMF/triethylamine

Cyclo(D-Trp-(ε-2-Cl-CBZ)Lys-(OBZl)Thr-Phe-Pro-Phe)

↓ HF/Anisole

Cyclo-(D-Trp-Lys-Thr-Phe-Pro-Phe)

The following Examples are given to illustrate the methods used to carry out the present invention. It is to be understood that these Examples are given for purposes of illustration and not limitation.

Example 1

Preparation of D-Trp-(ε-2-Cl-CBZ)Lys-(OBZl)Thr-Phe-Pro-Phe-OCH$_2$Ø-resin

Chloromethyl resin (2% cross-linked Merrifield resin, 862.0 g. (2.37 moles), having 2.75 meq. chlorine/g., and 607.0 g. (2.37 moles, 1 equivalent) of BOC-Phe were added to 4320 ml. of peroxide-free tetrahydrofuran. The mixture was stirred in an oil bath at 80°C bath temperature for 45 minutes. Triethylamine, 310.0 ml., was added and the reaction mixture stirred at 80°C bath temperature for 70 hours, cooled to 25°C and transferred to a stirred solid phase reaction column with 2000 ml. of tetrahydrofuran. After removal of the solvent, the resin was washed using the stirred column with:

3 x 2000 ml. of tetrahydrofuran
4 x 5170 ml. of ethanol
1 x 5170 ml. of acetic acid
3 x 5170 ml. of water
3 x 5170 ml. of methanol
3 x 5170 ml. of chloroform

The BOC-Phe-O—CH$_2$Ø-resin was dried *in vacuo* at 25°C for 16 hours, giving 1203 g. of BOC-Phe-O—CH$_2$Ø-resin containing 1.2 mmole of phenylalanine/g. of resin.

BOC-Phe-O—CH$_2$Ø-resin (2.13 g.; 2.0 mmole) was carried through the procedures in Tables III and IV using 2 deblockings (2 minutes and 25 minutes) with 25% TFA in methylene chloride and 2.5 equivalents of BOC-amino acid in the required sequence until the desired BOC-hexapeptide-O—CH$_2$Ø-resin was obtained.

DCCI was used as the sole coupling agent in every step.

The coupling of each amino acid proceeded smoothly. Best yields were obtained when the coupling was repeated in each step. When the coupling was repeated, the initial two chloroform washes, the deblocking step and the succeeding three chloroform washes were all omitted and replaced by a single chloroform wash.

The coupling reactions were carried out in methylene chloride, freshly degassed DMF or a mixture of these two solvents. The N-terminal amino group was blocked with a BOC group in each case; the hydroxy group of Thr was blocked with Bzl and the ε-amino group of Lys with 2-Cl-CBZ.

When the desired BOC-hexapeptide-O—CH$_2$Ø-resin was obtained, the N-terminal BOC group was removed by the terminal deblocking procedure set forth in Table V.

TABLE III

| Solvent or reagent (number of treatments or washes) | $CHCl_3$ (2) | 25% TFA in $CH_2Cl_2$ (2) | $CHCl_3$(3) | $NEt_3$-$CH_2Cl_2$ (1:9) (2) | $CHCl_3$(3) $CH_2Cl_2$(3) | BOC AA in $CH_2Cl_2$ DMF or a mixture of both | 0.5M DCCI in $CH_2Cl_2$ | DMF(1) MeOH(1) DMF(1) MeOH(1) $CHCl_3$(2) |
|---|---|---|---|---|---|---|---|---|
| Vol. in ml. | 40 | 20 | 40 | 40 | 40 | 25 | 10 | 40 |
| Time in min. | 5 | 2 and 25 | 2 | 5 and 5 | 2 | 5 | 5 coupling 30 | 2 |

TABLE IV

| Protected Amino Acid | Solvent Ml. |
|---|---|
| I | |
| BOC—N-Me-Ala (1.08 g.) | 20 ml. $CH_2Cl_2$ |
| Recouple | |
| BOC-Phe (1.32 g.) | 20 ml. $CH_2Cl_2$ |
| Recouple | |
| BOC (OBzl)Thr (1.55 g.) | 20 ml. $CH_2Cl_2$ |
| Recouple | |
| BOC-($\varepsilon$-2-Cl-CBZ)Lys (2.24 g.) | 20 ml. $CH_2Cl_2$ |
| Recouple | |
| BOC—D-Trp (1.52 g.) | 15 ml. $CH_2Cl_2$, 5 ml. DMF |
| Recouple | |
| II | |
| BOC-Pro (1.80 g.) | 25 ml. $CH_2Cl_2$ |
| Recouple | |
| BOC-Phe (1.32 g.) | 25 ml. $CH_2Cl_2$ |
| Recouple | |
| BOC (OBZ)Thr (1.55 g.) | 25 ml. $CH_2Cl_2$ |
| Recouple | |
| BOC-(2-Cl-CBZ)Lys (2.24 g.) | 25 ml. $CH_2Cl_2$ |
| Recouple | |
| BOC—D-Trp (1.52 g.) | 20 ml. $CH_2Cl_2$, 5 ml. DMF |
| Recouple | |

TABLE V

| | TERMINAL DEBLOCKING PROGRAM | | | |
|---|---|---|---|---|
| Solvent or reagent | $CHCl_3$(1) | 25% TFA in $CH_2Cl_2$+1% | $CHCl_3$ | MeOH(2) $CH_2Cl_2$(1) |
| (number of treatments or washes) | | Ethanedithiol (2) | (3) | MeOH(2) $CH_2Cl_2$(2) |
| Vol. in ml. | 40 | 40 | 40 | 40 |
| Time in minutes | 5 | 2 and 25 | 2 | 2 |

After the procedures of Tables III, IV and V were completed, the blocked hexapeptide-$OCH_2Ø$-resin is dried overnight and weighs 3.7 g.

Example 2

Preparation of D-Trp-(ε-2-Cl-CBZ)Lys-(OBZl)Thr-Phe-N-Me-Ala-Phe-$NHNH_2$

The resin from Example 1 is combined with 30 ml. of a 2:1 mixture of dimethylacetamide and hydrazine and stirred at room temperature for 1 hour. The insoluble resin is removed by filtration and the solution is evaporated to remove the dimethylacetamide. The residue is placed under high vacuum overnight to remove all volatile materials. A foam resulted from dissolving the residue in methanol, filtering and evaporating to dryness, weighing 2.19 g.

Example 3

Preparation of D-Trp-(ε-2-Cl-CBZ)Lys-(OBZl)Thr-Phe-N-Me-Ala-Phe-$N_3$

The foam from Example 2 is combined with 15 ml of degassed dimethylformamide under a blanket of nitrogen and cooled to −10°C, and 5 equivalents of 4.52 M. hydrogen chloride in tetrahydrofuran (2.2 ml.) is added. The solution is cooled to −25°C and 5 ml. of a 1:19 mixture of isoamyl nitrite in dimethylformamide is added in portions until a positive starch/KI test is obtained. The completion of the reaction is followed by thin layer chromatography and the disappearance of the hydrazide starting material.

Example 4

Preparation of Cyclo(D-Trp-(ε-2-Cl-CBZ)Lys-(O-BZl)Thr-Phe-N-Me-Ala-Phe)

The azide compound from Example 3 is added to 600 ml. of degassed dimethylformamide, precooled to −25°C, the pH adjusted to 8, with triethylamine, and the reaction mixture placed in the freezer overnight. The pH is readjusted to 8 if necessary after about 14 hours and the mixture stored for 16 hours at −20°C and 16 hours at 5°C. Thin layer chromatography indicates that the reaction is completed. The mixture is concentrated to dryness, dissolved in 150 ml. of a 3:1 dimethylformamide/water mixture and treated with a mixed bed anioncation exchange resin for 1 hour. The mixture is filtered and concentrated to dryness *in vacuo* to an oil, and the residue is triturated with methanol, affording 1.91 g. of a white solid.

Example 5

Preparation of Cyclo(D-Trp-Lys-Thr-Phe-N-Me-Ala-Phe)

1.91 G. (1.8 mmoles) of the protected cyclic hexapeptide of Example 4 is combined in a teflon lined chamber with 2 ml. of anisole. The chamber is then evacuated and filled with liquid hydrogen fluoride at the temperature of the dry ice/acetone bath. The temperature is raised to 0°C and stirring continued for 1 hour. The hydrogen fluoride is allowed to evaporate and the residue placed *in vacuo* until a slurry is formed. The slurry is triturated with ethyl acetate and filtered affording 1.29 g. of a fine powder. The powder is placed on 300 g. of silica gel and eluted with a 10:5:1:3 mixture of EPAW, eluting with 9 ml. fractions. Fractions 55—66 are combined and concentrated. The product is precipitated from a mixture of chloroform, ethylacetate and hexane affording 679 mg.

Example 6

Preparation of D-Trp-(ε-2-Cl-CBZ)Lys-(OBZl)Thr-Phe-Pro-Phe-$NHNH_2$

The resin from Example 1 is combined with 30 ml. of a 2:1 mixture of dimethylacetamide and hydrazine and stirred at room temperature for 1 hour. The insoluble resin is removed by filtration and the solution is evaporated to remove the dimethylacetamide. The residue is placed under high vacuum overnight to remove all volatile materials. A foam resulted from dissolving the residue in methanol, filtering and evaporating to dryness, weighing 1.84 g.

Example 7

Preparation of D-Trp-(ε-2-Cl-CBZ)Lys-(OBZl)Thr-Phe-Pro-Phe-$N_3$

The foam from Example 2 is combined with 15 ml. of degassed dimethylformamide under a blanket of nitrogen and cooled to −10°C, and 5 equivalents of 5.8 M. hydrogen chloride in tetrahydrofuran (1.7 ml.) is added. The solution is cooled to −25°C and 5 ml. of a 1:19 mixture of isoamyl nitrite in dimethylformamide is added. The completion of the reaction is followed by thin layer chromatography and the disappearance of the hydrazide starting material.

Example 8

Preparation of Cyclo(D-Trp-(ε-2-Cl-CBZ)Lys-(OBZl)Thr-Phe-Pro-Phe)

The azide compound of Example 3 is added to 600 ml. of degassed dimethylformamide, precooled to −25°C, the pH adjusted to 8, and the reaction mixture placed in the freezer overnight. The pH is readjusted to 8 if necessary after about 14 hours and the mixture stored for 16 hours at −20°C and 16 hours at 5°C. Thin layer chromatography indicates that the reaction is completed. The mixture is concentrated to dryness dissolved in 150 ml. of a 3:1 dimethylformamide/water mixture and treated with a mixed bed anion-cation exchange resin for 2 hours. The mixture is filtered and concentrated to dryness *in vacuo,* and the residue is dissolved in methanol, filtered and evaporated to dryness affording a foam.

Example 9

Preparation of Cyclo(D-Trp-Lys-Thr-Phe-Pro-Phe)

2.13 G. (2 mmoles) of the protected cyclic hexapeptide of Example 4 is combined in a teflon lined chamber with 2 ml. of anisole. The chamber is then evacuated and filled with liquid hydrogen fluoride at the temperature of the dry ice/acetone bath. The temperature is raised to 0°C and stirring continued for 1 hour. The hydrogen fluoride is allowed to evaporate and the residue placed *in vacuo* until a slurry is formed. The slurry is treated with ethyl acetate and filtered affording 1.19 g. of a fine powder. 300 Mg. of this powder is placed on a 1000 μ silica gel preparative layer chromatography plate and eluted with a 70:30:5 mixture of chloroform, methanol and concentrated aqueous ammonia, affording 200 mg. of product. The remaining crude product is placed on a silica gel column, and eluted with the same solvent. The presence of the product in the eluent is determined by thin layer chromatographic analysis and, after freeze drying, affords about 510 mg. of product.

Following the above procedure, and by modifying only the selection and order of amino acids in the process of Example 1, there are prepared other cyclic hexapeptides of this invention.

The instant cyclic hexapeptide analogs of somatostatin are tested and compared with the effects of somatostatin in an *in vitro* test for the inhibition of growth hormone. The test is described as follows:

"Rat pituitaries were isolated according to the procedures of Vale and Grant "In vitro Pituitary Hormone Secretion Assay for Hypophysiotropic Substances" in Methods in Enzymology. Vol. XXXVII, eds. O'Malley, B. W. and Hardman, J. G. (Academic Press, Inc., New York) pp.5—93 (1975).

After 4 days in culture, the cells were washed and incubated for 4 hours in Dulbecco-modified Eagle's medium in the presence or absence of graded doses of each analog or somatostatin. The medium was then collected for subsequent growth hormone determination by a double antibody radioimmunoassay for rat growth hormone."

The tert results for some of the compounds of this invention are recorded below with the results for somatostatin listed first and given the arbitrary value of 1. The results for the instant compounds are given as multiples or fractions of the effect of somatostatin. The numbers in parentheses are the finducial limits for the number preceding. The first of the instant compounds listed is the compound prepared in Examples 1—5. The compound is written slightly different, however, to conform to the order of the amino acids found in somatostatin.

Activity of Cyclichexapeptide Analogs of Somatostatin.

| Compound | Growth Hormone Release Inhibition In Vitro |
|---|---|
| Somatostatin | 1 |
| Cyclo(N-Me-Ala-Phe-D-Trp-Lys-Thr-Phe) | 4.38 (2.15—11.90) |
| Cyclo(N-Me-D-Ala-Phe-D-Trp-Lys-Thr-Phe) | 2.13 (0.83—6.00) |
| Cyclo(Pro-Phe-D-Trp-Lys-Thr-Phe) | 1.71 (1.31—2.32) |
| Cyclo(Pro-Phe-L-Trp-Lys-Thr-Phe) | 1.59 (1.33—1.92) |
| Cyclo(Pro-Phe-D-5F-Trp-Lys-Thr-Phe) | 2.4 (2.1—2.8) |
| Cyclo(Pro-Phe-L-5F-Trp-Lys-Thr-Phe) | 2.5 (1.9—3.2) |
| Cyclo(Pro-Phe-L-5-$OCH_3$-Trp-Lys-Thr-Phe | 0.17 (0.12—0.25) |
| Cyclo(Pro-Phe-D-Trp-Orn-Thr-Phe) | 0.16 (0.15—0.17) |
| Cyclo(Pro-Phe-D-Trp-Lys-Ser-Phe) | 1.4 (1.1—1.7) |
| Cyclo(Pro-Phe-D-Trp-Lys-$\alpha$-ABU-Phe) | 0.82 (0.57—1.18) |
| Cyclo(Pro-Phe-D-Trp-Lys-Thr-Leu) | 0.16 (0.07—0.33) |
| Cyclo(Pro-Phe-D-Trp-Lys-Thr-p-Cl-Phe) | 0.86 (0.75—1.0) |
| Cyclo(Pro-p-Cl-Phe-D-Trp-Lys-Thr-Phe) | 1.14 (0.98—1.34) |
| Cyclo(Pro-Leu-D-Trp-Lys-Thr-Phe) | 0.29 (0.13—0.57) |
| Cyclo(Pro-Ala-D-Trp-Lys-Thr-Phe) | 0.19 (0.1—0.38) |
| Cyclo(Pro-Tyr-D-Trp-Lys-Thr-Phe) | 14.3 (8.3—33.0) |

The effects of the instant cyclichexapeptide analogs of somatostatin are determined by the following procedure.

Compounds were tested for their ability to inhibit pentagastrin evoked gastric secretion in the chronic fistula dog. Female beagle dogs with a chronic gastric fistula were given pentagastrin (2.5$\mu$g./kg./hour, i.v. from −60 to 120 min.) and gastric outputs were collected via the fistula cannula. Samples were analyzed at 30 minute intervals for volume (ml.) and titratable acid (mEq/L) (Titration to pH 7 with 0.01N NaOH); total acid output (mEq) was calculated as the production of output volume and acid concentrations. Test compounds were infused at a constant rate from 0 to 60 minutes. Data have been expressed as percent change of total acid output relative to a placebo trial in the same animals.

In the following data, the results for somatostatin are given first for comparison purposes.

Effects of Cyclichexapeptide Analogs of Somatostatin on Gastric Secretion
(Dose 0.8 μg/ml./min.—infusion 0—60 min.)

| Compound | Gastric Secretion % Inhibition | | | | | | | |
|---|---|---|---|---|---|---|---|---|
| | Vol. | | | | Acid Concentration | | | |
| | 0—30 | 30—60 | 60—90 | 90—120 | 0—30 | 30—60 | 60—90 | 90—120 |
| somatostatin | 85 | 97 | 81 | 37 | 16 | 77 | 61 | 14 |
| Cyclo(N-Me-Ala-Phe-D-Trp-Lys-Thr-Phe) | 91 | 92 | 90 | 84 | 15 | 41 | 47 | 48 |
| Cyclo(N-Me-D-Ala-Phe-D-Trp-Lys-Thr-Phe) | 50 | 84 | 0 | 0 | 12 | 34 | 9 | 0 |
| Cyclo(Pro-Phe-D-Trp-Lys-Thr-Phe) | 88 | 98 | 95 | 86 | 18 | 47 | 54 | 68 |
| Cyclo(Pro-Phe-D-Trp-Lys-Thr-p-Cl-Phe) | 88 | 88 | 85 | 44 | 52 | 82 | 94 | 38 |
| Cyclo(Pro-p-Cl-Phe-D-Trp-Lys-Thr-Phe) | 79 | 94 | 51 | 25 | 30 | 53 | 24 | 12 |
| Cyclo(Pro-Tyr-D-Trp-Lys-Thr-Phe) | 67 | 87 | 87 | 64 | 30 | 49 | 52 | 27 |

**Claims for the Contracting States: BE CH DE FR GB IT LI LU NL SE**

1. A compound having the formula:

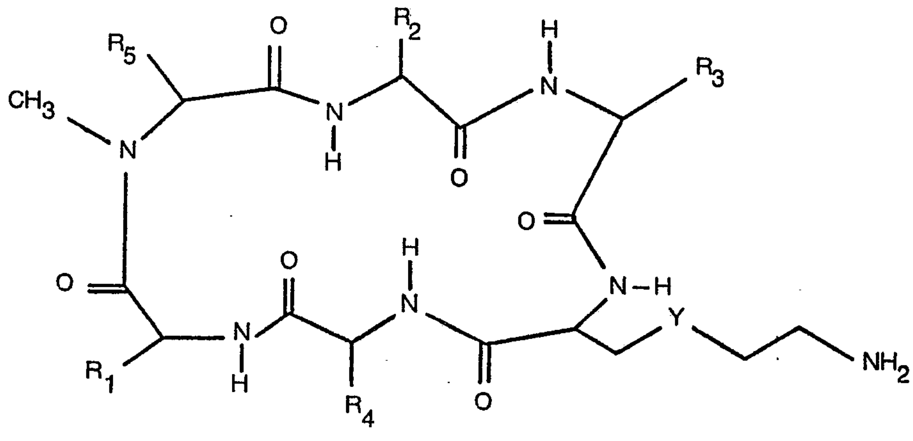

I

or

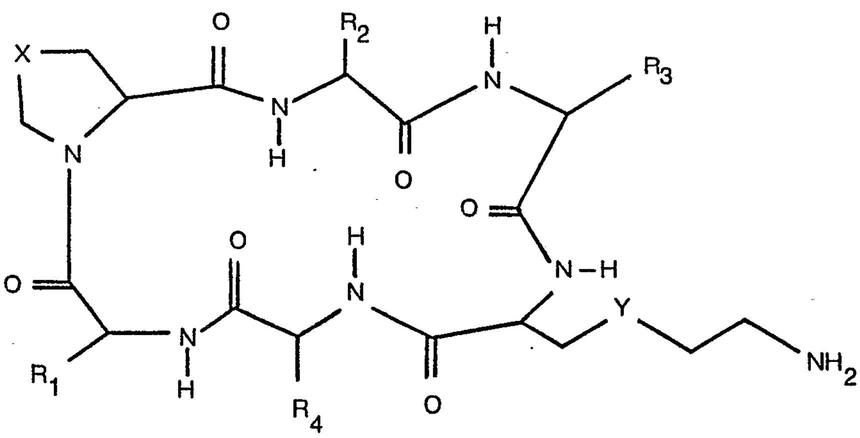

II

in which X is $(CH_2)_n$ where $n$ is 0, 1 or 2, or X is sulfur;

Y is $(CH_2)_m$ where $m$ is 0, 1 or 2, or Y is sulfur such that the sulfur may be in any position along the chain;

each of $R_1$ and $R_2$, independently of the other, is $C_{1-5}$ alkyl, benzyl, substituted benzyl in which the substituent is one or two of $C_{1-5}$alkyl, halogen, hydroxy, amino, nitro and $C_{1-5}$ alkoxy; or $C_{1-5}$ alkyl substituted with a 5- or 6-membered heterocyclic ring;

$R_3$ is 3-indolylmethyl or substituted 3-indolymethyl in which the substituent is $C_{1-5}$ alkyl, $C_{1-5}$ alkoxy or halogen;

$R_4$ is $C_{1-5}$ alkyl, $C_{1-5}$ hydroxyalkyl, benzyl, carboxy ($C_{1-5}$ alkyl), $C_{1-5}$ aminoalkyl or substituted benzyl where the substituent is $C_{1-5}$ alkyl, $C_{1-5}$ alkoxy, hydroxy, halogen, amino or nitro; and

$R_5$ is $C_{1-5}$ alkyl, benzyl, or substituted benzyl where the substituent is $C_{1-5}$ alkyl, $C_{1-5}$ alkoxy, hydroxy, halogen, amino or nitro.

2. A compound as claimed in claim 1 in which X and Y are both methylene; $R_1$ and $R_2$ are as defined in claim 1; $R_3$ is 3-indolylmethyl or substituted indolylmethyl where the substituent is methoxy or fluoro; $R_4$ is methyl, ethyl, hydroxymethyl or hydroxyethyl; and $R_5$ is methyl.

3. A compound as claimed in claim 2 in which $R_3$ is 3-indolylmethyl and $R_4$ is hydroxyethyl.

4. Cyclo (Pro-Tyr-D-Trp-Lys-Thr-Phe).

5. Cyclo (Pro-Phe-D-Trp-Lys-Thr-Phe).

6. Cyclo (N-Me-Ala-Tyr-D-Trp-Lys-Thr-Phe).

7. A process for preparing a cyclic hexapeptide compound as claimed in claim 1 comprising:

(a) preparing a corresponding blocked linear peptide attached to a solid phase resin;

(b) selectively deblocking the N-terminal amine group;

(c) removing the linear peptide from the resin;

(d) treating the linear peptide with a cyclizing agent to form the amide bond of the desired cyclic hexapeptide;

(e) removing the side-chain-protecting groups.

8. A process as claimed in claim 7 in which step (c) comprises treating the linear peptide resin with hydrazine to form the hydrazide.

9. A cyclic hexapeptide as claimed in claim 1 for use in inhibiting the release of growth hormone in an animal.

10. A pharmaceutical composition comprising a cyclic hexapeptide as claimed in claim 1 or a non-toxic acid-addition salt thereof in a pharmaceutically acceptable liquid or solid carrier.

**Claims for the Contracting State: AT**

1. A process for preparing a cyclic hexapeptide compound having the formula:

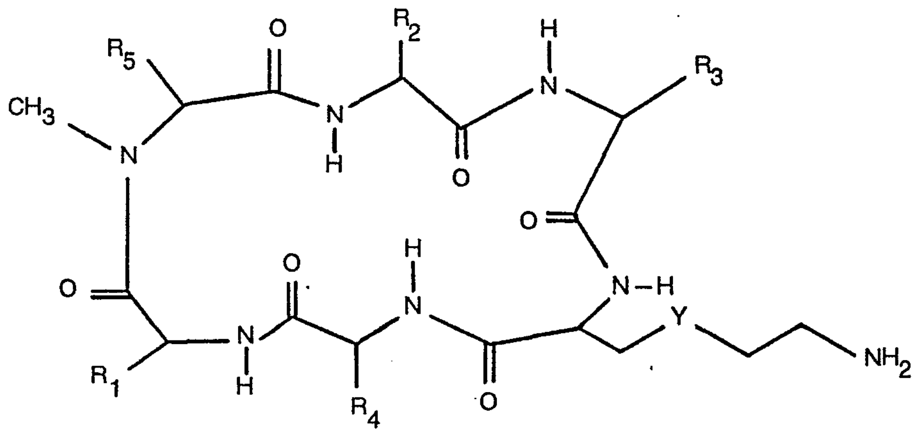

I

or

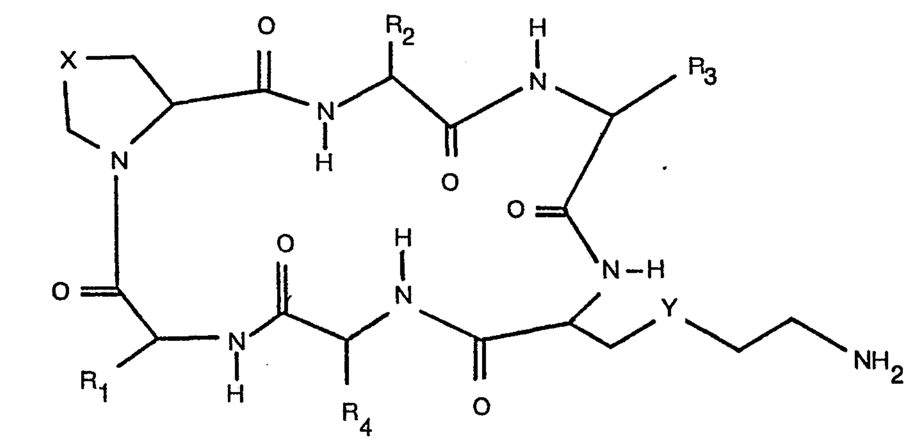

II

in which X is $(CH_2)_n$ where $n$ is 0, 1 or 2, or X is sulfur;

Y is $(CH_2)_m$ where $m$ is 0, 1 or 2, or Y is sulfur such that the sulfur may be in any position along the chain;

each of $R_1$ and $R_2$, independently of the other, is $C_{1-5}$ alkyl, benzyl, substituted benzyl in which the substituent is one or two of $C_{1-5}$ alkyl, halogen, hydroxy, amino, nitro and $C_{1-5}$ alkoxy; or $C_{1-5}$ alkyl substituted with a 5- or 6-membered heterocyclic ring;

$R_3$ is 3-indolylmethyl or substituted 3-indolymethyl in which the substituent is $C_{1-5}$ alkyl, $C_{1-5}$ alkoxy or halogen;

$R_4$ is $C_{1-5}$ alkyl, $C_{1-5}$ hydroxyalkyl, benzyl, carboxy ($C_{1-5}$ alkyl), $C_{1-5}$ aminoalkyl or substituted benzyl where the substituent is $C_{1-5}$ alkyl, $C_{1-5}$ alkoxy, hydroxy, halogen, amino or nitro; and

$R_5$ is $C_{1-5}$ alkyl, benzyl, or substituted benzyl where the substituent is $C_{1-5}$ alkyl, $C_{1-5}$ alkoxy, hydroxy, halogen, amino or nitro, comprising:

(a) preparing a corresponding blocked linear peptide attached to a solid phase resin;

(b) selectively deblocking the N-terminal amine group;

(c) removing the linear peptide from the resin;

(d) treating the linear peptide with a cyclizing agent to form the amide bond of the desired cyclic hexapeptide;

(e) removing the side-chain-protecting groups.

2. A process as claimed in Claim 1 in which step (c) comprises treating the linear peptide resin with hydrazine to form the hydrazide.

3. A process as claimed in Claim 1 or 2 in which, in the formula, X and Y are both methylene; $R_1$ and $R_2$ are as defined in Claim 1; $R_3$ is 3-indolylmethyl or substituted indolylmethyl where the substituent is methoxy or fluoro; $R_4$ is methyl, ethyl, hydroxymethyl or hydroxyethyl; and $R_1$ is methyl.

4. A process as claimed in Claim 3 in which, in the formula, $R_3$ is 3-indolylmethyl and $R_4$ is hydroxyethyl.

5. A process as claimed in Claim 1 or 2 as applied to the preparation of cyclo (Pro-Tyr-D-Trp-Lys-Thr-Phe).

6. A process as claimed in Claim 1 or 2 as applied to the preparation of cyclo (Pro-Phe-D-Trp-Lys-Thr-Phe).

7. A process as claimed in Claim 1 or 2 as applied to the preparation of cyclo(N-Me-Ala-Tyr-D-Trp-Lys-Thr-Phe).

**Revendications pour les Etats contractants: BE CH LI DE FR GB IT LU NL SE**

1. Un composé ayant pour formule:

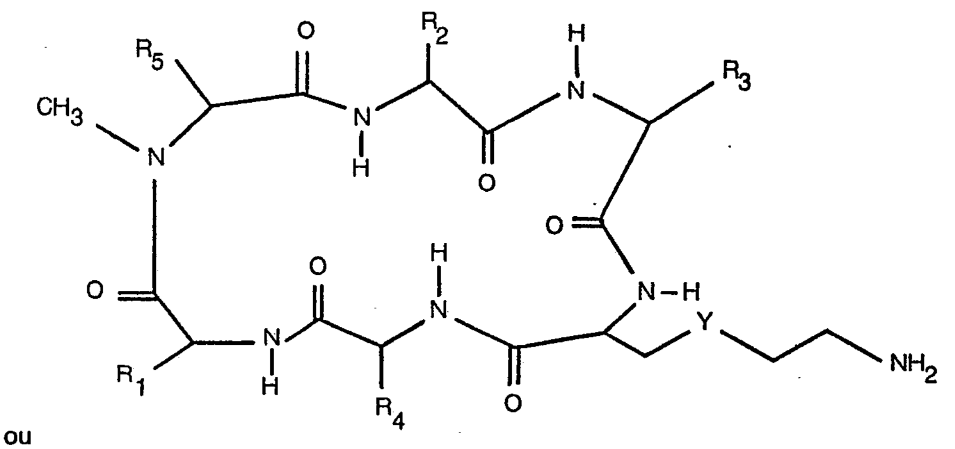

I

ou

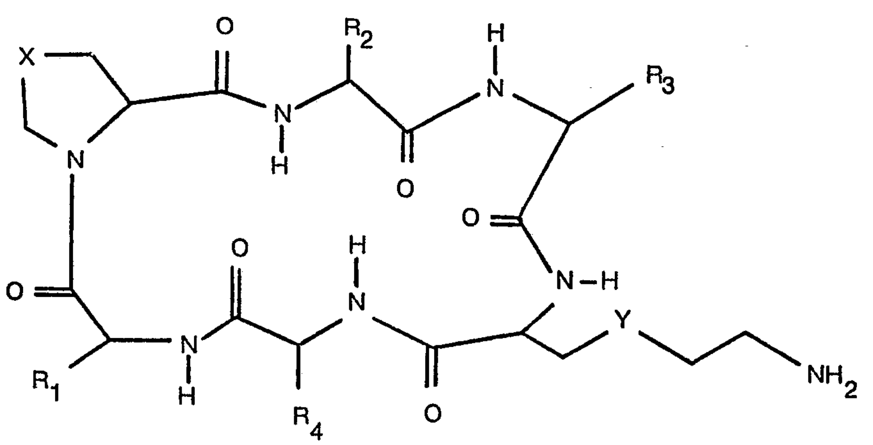

II

où X est $(CH_2)_n$, où n est 0, 1 ou 2 ou X est le soufre: Y est $(CH_2)_m$ où m est 0, 1 ou 2 ou Y est le soufre si bient que le soufre peut être en une position quelconque le long de la chaîne;

Chacun de $R_1$ et $R_2$ indépendamment de l'autre est un alkyle en $C_{1-5}$, un benzyle, un benzyle substitué dont le substituant est un ou deux alkyles en $C_{1-5}$, halogène, hydroxy, amino, nitro et alcoxy en $C_{1-5}$; ou un alkyle en $C_{1-5}$ substitué par un hétérocycle pentagonal ou hexagonal;

$R_3$ et 3-indolylméthyle ou un 3-indolylméthyle substitué dont le substituant est un alkyle en $C_{1-5}$, un alcoxy en $C_{1\div5}$ ou un halogène;

$R_4$ est un alkyle en $C_{1-5}$, un hydroxyalkyle en $C_{1-5}$, un benzyle, un carboxy-(alkyle $C_{1-5}$), un aminoalkyle en $C_{1-5}$ ou un benzyle substitué dont le substituant est un alkyle en $C_{1-5}$, un alcoxy en $C_{1-5}$, un hydroxy, un halogène, un amino ou un nitro; et

$R_5$ est un alkyle en $C_{1-5}$, un benzyle ou un benzyle substitué dont le substituant est un alkyle en $C_{1-5}$, un alcoxy en $C_{1-5}$, un hydroxy, un halogène, un aminon ou un nitro.

2. Un composé comme revendiqué dans la revendication 1, dans lequel X et Y sont tous deux un méthylène; $R_1$ et $R_2$ sont comme définis dans la revendication 1; $R_3$ est un 3-indolylméthyle ou un 3-

indolylméthyle substitué dont le substituant est un méthoxy ou un fluoro; $R_4$ est un méthyle, un éthyle, un hydroxyméthyle ou un hydroxyéthyle; et $R_5$ et un méthyle.

3. Un composé selon la revendication 2, dans lequel $R_3$ est un 3-indolylméthyle et $R_4$ est un hydroxyéthyle.

4. Cyclo (Pro-Tyr-D-Trp-Lys-Thr-Phé).

5. Cyclo (Pro-Phé-D-Trp-Lys-Thr-Phé).

6. Cyclo (N-Mé-Ala-Tyr-D-Trp-Lys-Thr-Phé).

7. Un procédé pour préparer un hexapeptide cyclique comme revendiqué dans la revendication 1, comprenant:

(a) la préparation d'un peptide linéaire bloqué correspondant attaché à une phase solide de résine;

(b) le déblocage sélectif du radical amine N-terminal;

(c) la séparation du peptide linéaire de la résine;

(d) le traitement du peptide linéaire avec un agent cyclisant pour former la liaison amide de l'hexapeptide cyclique désiré;

(e) l'élimination des groupes protecteurs latéraux.

8. Procédé comme revendiqué dans la revendication 7, dans lequel le stade (c) comprend le traitement de peptide linéaire-résine avec l'hydrazine pour former l'hydrazide.

9. Un hexapeptide cyclique comme revendiqué dans la revendication 1, pour l'emploi dans l'inhibition de la libération de l'hormone de croissance chez un animal.

10. Une composition pharmaceutique comprenant un hexapeptide cyclique comme revendiqué dans la revendication 1 ou un de ses sels d'addition d'acide non toxique dans un véhicule liquide ou solide acceptable en pharmacie.

**Revendications pour l'Etat contractant: AT**

1. Procédé pour préparer un hexapeptide cyclique ayant pour formule:

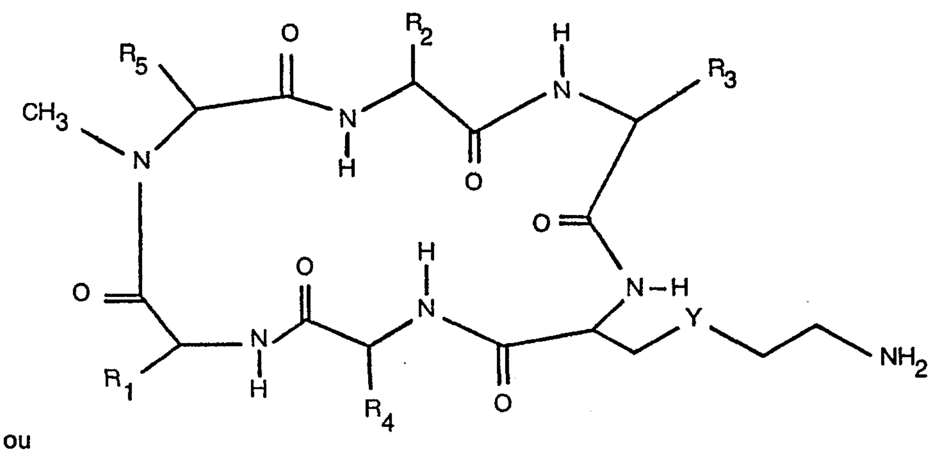

I

ou

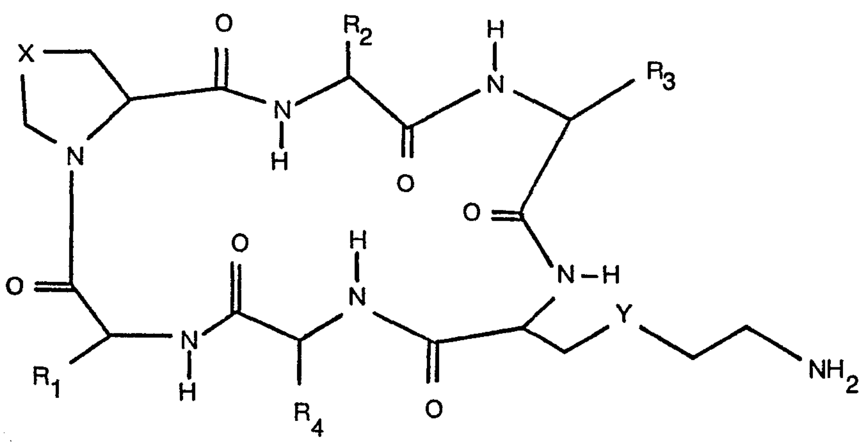

II

où X est $(CH_2)_n$ où n est 0, 1 ou 2 ou X est le soufre: Y est $(CH_2)_m$ où m est 0, 1 ou 2 ou Y est le soufre si bien que le soufre peut être en une position quelconque le long de la chaîne;

Chacun de $R_1$ et $R_2$ indépendamment de l'autre est un alkyle en $C_{1-5}$, un benzyle, un benzyle substitué dont le substituant est un ou deux alkyles en $C_{1-5}$, halogène, hydroxy, amino, nitro et alcoxy en $C_{1-5}$; ou un alkyle en $C_{1-5}$ substitué par un hétérocycle pentagonal ou hexagonal;

$R_3$ et 3-indolylméthyle ou un 3-indolylméthyle substitué dont le substituant est un alkyle en $C_{1-5}$, un alcoxy en $C_{1\div5}$ ou un halogène;

$R_4$ est un alkyle en $C_{1-5}$, un hydroxyalkyle en $C_{1-5}$, un benzyle, un carboxy(alkyle $C_{1-5}$), un aminoalkyle en $C_{1-5}$ ou un benzyle substitué dont le substituant est un alkyle en$C_{1-5}$, un alcoxy en $C_{1-5}$, un hydroxy, un halogène, un amino ou un nitro; et

$R_5$ est un alkyle en $C_{1-5}$, un benzyle ou un benzyle substitué dont le substituant est un alkyle en $C_{1-5}$, un alcoxy en $C_{1-5}$, un hydroxy, un halogène, un amino ou un nitro, qui comprend:

(a) la préparation d'un peptide linéaire à bloqué correspondant fixé à une phase solide de résine;

(b) le déblocage sélectif du radical amine N-terminal

(c) la séparation du peptide linéaire de la résine;

(d) le traitement de peptide linéaire avec un agent de cyclisation pour former la liaison amide de l'hexapeptide cyclique désiré;

(e) l'élimination des groupes protecteurs latéraux.

2. Un procédé comme revendiqué dans la revendication 1, dans lequel le stade (c) comprend le traitement du peptide linéaire-résine avec l'hydrazine pour former l'hydrazine.

3. Un procédé comme revendiqué dans la revendication 1 ou 2, dans lequel, dans la formule X et Y sont tous deux un méthylène; $R_1$ et $R_2$ sont comme défini dans la revendication 1; $R_3$ est un 3-indolyméthyle ou un 3-indolylméthyle substitué dont le substituant est un méthoxy ou un fluoro; $R_4$ est un méthyle, en éthyle, un hydroxyméthyle ou un hydroxyéthyle; et $R_5$ est un méthyle.

4. Procédé comme revendiqué dans la revendication 3, dans lequel, dans la formule $R_3$ est un 3-indolylméthyle et $R_4$ est un hydroxyéthyle.

5. Un procédé comme revendiqué dans la revendication 1 ou 2, appliqué à la préparation du cyclo (Pro-Tyr)D-Trp-Lys-Thr-Phé).

6. Un procédé comme revendiqué dans la revendication 1 ou 2, appliqué à la préparation du cyclo (Pro-Phé-D-Trp-Lys-Thr-Phé).

7. Un procédé comme revendiqué dans la revendication 1 ou 2 appliqué à la préparation du cyclo (N-Mé-Ala-Tyr-D-Trp-Lys-Thr-Phé).

**Patentansprüche für die Vertragsstaaten: BE CH LI DE FR GB IT LU NL SE**

1. Verbindungen der Formel

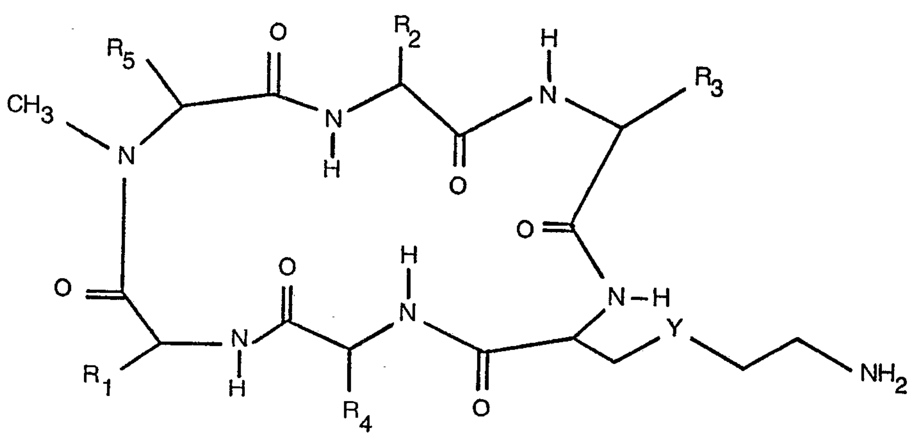

I

oder

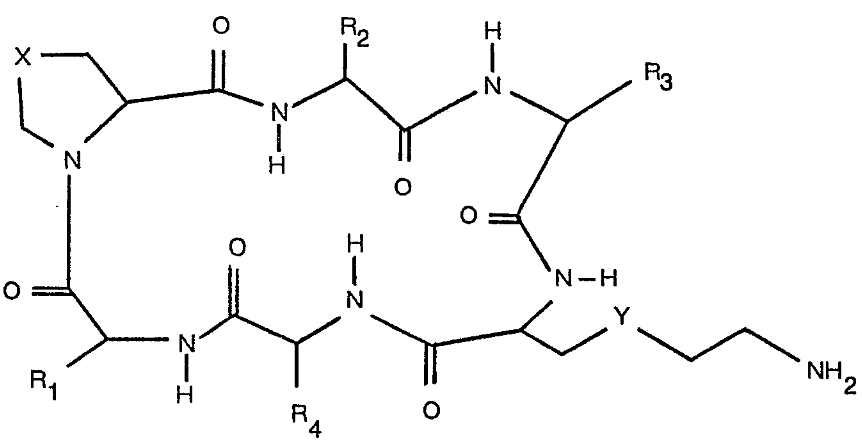

II

in der

X $(CH_2)_n$ bedeutet, wobei n 0, 1 oder 2 ist, oder X Schwefel bedeutet;

Y $(CH_2)_m$ bedeutet, wobei m 0, 1 oder 2 ist, oder Y Schwefel bedeutet, wobei sich der Schwefel an einer beliebigen Stelle entlang der Kette befinden kann;

$R_1$ und $R_2$ jeweils unabhängig voneinander $C_{1-5}$-Alkyl, Benzyl, substituiertes Benzyl, wobei es sich bei dem Substituenten um einen oder zwei der Reste $C_{1-5}$-Alkyl, Halogen, Hydroxy, Amino, Nitro und $C_{1-5}$-Alkoxy handelt, oder durch einen 5- oder 6-gliedrigen heterocyclischen Ring substituiertes $C_{1-5}$-Alkyl bedeuten;

$R_3$ 3-Indolylmethyl oder substituiertes 3-Indolylmethyl bedeutet, wobei es sich bei dem Substituenten um $C_{1-5}$-Alkyl, $C_{1-5}$-Alkoxy oder Halogen handelt;

$R_4$ $C_{1-5}$-Alkyl, $C_{1-5}$-Hydroxyalkyl, Benzyl, Carboxy-$(C_{1-5})$-alkyl, $C_{1-5}$-Aminoalkyl oder substituiertes Benzyl, wobei es sich bei dem Substituenten um $C_{1-5}$-Alkyl, $C_{1-5}$-Alkoxy, Hydroxy, Halogen, Amino oder Nitro handelt, bedeutet; und $R_5$ $C_{1-5}$-Alkyl, Benzyl oder substituiertes Benzyl bedeutet, wobei es sich bei dem Substituenten um $C_{1-5}$-Alkyl, $C_{1-5}$-Alkoxy, Hydroxy, Halogen, Amino oder Nitro handelt.

2. Verbindungen nach Anspruch 1, dadurch gekennzeichnet, dass X und Y beide Methylen bedeuten, $R_1$ und $R_2$ die in Anspruch 1 definierte Bedeutung haben; $R_3$ 3-Indolylmethyl oder substituiertes Indolylmethyl bedeutet, wobei es sich bei dem Substituenten um Methoxy oder Fluor handelt; $R_4$ Methyl, Äthyl, Hydroxymethyl oder Hydroxyäthyl bedeutet; und $R_5$ Methyl bedeutet.

3. Verbindungen nach Anspruch 2, dadurch gekennzeichnet, dass $R_3$ 3-Indolylmethyl bedeutet und $R_4$ Hydroxyäthyl bedeutet.

4. Cyclo-(Pro-Tyr-D-Trp-Lys-Thr-Phe).

5. Cyclo-(Pro-Phe-D-Trp-Lys-Thr-Phe).

6. Cyclo-(N-Me-Ala-Tyr-D-Trp-Lys-Thr-Phe).

7. Verfahren zur Herstellung von cyclischen Hexapeptiden nach Anspruch 1, dadurch gekennzeichnet, dass man

(a) ein an ein Festphasenharz gebundenes entsprechendes blockiertes lineares Peptid herstellt;

(b) selektiv die N-terminale Amingruppe entblockiert;

(c) das lineare Peptid vom Harz entfernt;

(d) das linear Peptid mit einem Cyclisierungsmittel unter Bildung der Amidbindung des gewünschten cyclischen Hexapeptids behandelt;

(e) die Seitenkettenschutzgruppen entfernt.

8. Verfahren nach Anspruch 7, dadurch gekennzeichnet, dass die Stufe (c) die Behandlung des linearen Peptids mit Hydrazin unter Bildung des Hydrazids umfasst.

9. Cyclisches Hexapeptid nach Anspruch 1 zur Verwendung bei der Hemmung der Freisetzung von Wachstumshormon bei Tieren.

10. Arzneimittel, enthaltend ein cyclisches Hexapeptid nach Anspruch 1 oder nicht-toxisches Säureadditionssalz davin in einem pharmakologisch verträglichen festen oder flüssigen Träger.

**Patentansprüche für den Vertragsstaat: AT**

1. Ein Verfahren zur Herstellung einer cyclischen Hexapeptidverbindung mit der Formel:

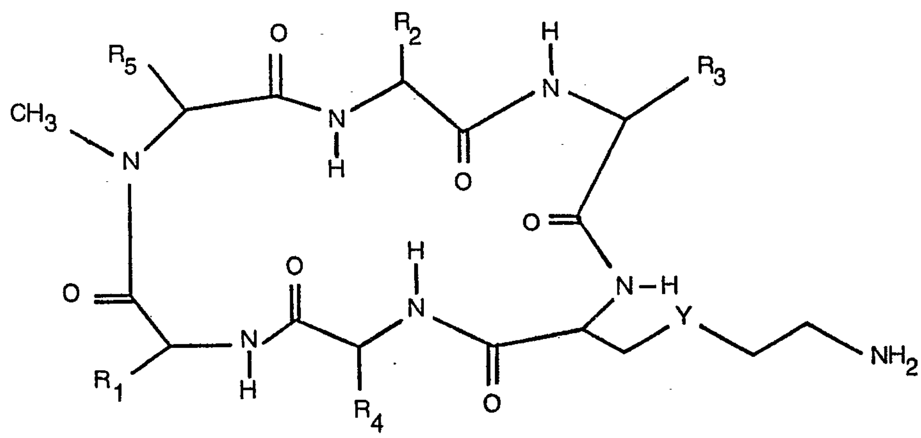

I

oder

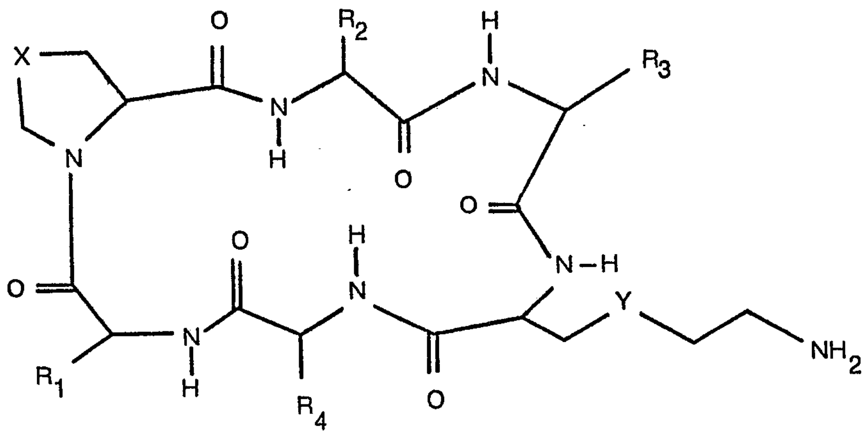

II

worin

X $(CH_2)_n$ ist worin n 0, 1 oder 2 ist, oder X Schwefel ist;

Y $(CH_2)_m$ ist, worin m 0, 1 oder 2 ist, oder Y Schwefel ist, wobei der Schwefel in irgendeiner Stellung längs der Kette vorliegen kann;

jedes $R_1$ und $R_2$ unabhängig vom anderen $C_{1-5}$Alkyl, Benzyl, substituiertes Benzyl, worin der Substituent ein oder zwei $C_{1-5}$Alkyl-, Halogen-, Hydroxy-, Amino-, Nitro- und $C_{1-5}$Alkoxyreste ist; oder $C_{1-5}$Alkyl, das durch einen 5-gliedrigen oder 6-gliedrigen heterocyclischen Ring substituiert ist, bedeutet;

$R_3$ 3-Indolylmethyl oder substituiertes 3-Indolylmethyl ist, worin der Substituent $C_{1-5}$Alkyl, $C_{1-5}$Alkoxy oder Halogen ist;

$R_4$ $C_{1-5}$Alkyl, $C_{1-5}$Hydroxyalkyl, Benzyl Carboxy($C_{1-5}$alkyl), $C_{1-5}$Aminoalkyl oder substituiertes Benzyl ist, worin der Substituent $C_{1-5}$Alkyl, $C_{1-5}$Alkoxy, Hydroxy, Halogen, Amino oder Nitro ist; und

$R_5$ $C_{1-5}$Alkyl, Benzyl oder substituiertes Benzyl ist, worin der Substituent $C_{1-5}$Alkyl, $C_{1-5}$Alkoxy, Hydroxy, Halogen, Amino oder Nitro ist, welches umfaßt:

(a) die Herstellung eines entsprechenden blockierten linearen Peptids, das an ein Festphasenharz gebunden ist;

(b) das selektive Entblockieren der N-endständigen Aminogruppe;

(c) die Entfernung des linearen Peptids von dem Harz;

(d) die Behandlung des linearen Peptids mit einem Cyclisierungsmittel unter Bildung der Amidbindung des gewünschten cyclischen Hexapeptids;

(e) die Entfernung der Seitemkettenschutzgruppen.

2. Ein Verfahren nach Anspruch 1, worin die Stufe (c) die Behandlung des linearen Peptidharzes mit Hydrazin unter Bildung des Hydrazids umfaßt.

3. Ein Verfahren nach Anspruch 1 oder 2, in welchem in der Formel I sowohl X als auch Y Methylen sind; $R_1$ und $R_2$ wie in Anspruch 1 definiert sind; $R_3$ 3-Indolylmethyl oder substituiertes Indolylmethyl ist, worin der Substituent Methoxy oder Fluor ist; $R_4$ Methyl, Ethyl, Hydroxymethyl oder Hydroxyethyl ist; und $R_1$ Methyl ist.

4. Ein Verfahren nach Anspruch 3, in welchem in der Formel $R_3$ 3-Indolylmethyl ist und $R_4$ Hydroxyethyl ist.

5. Ein Verfahren nach Anspruch 1 oder 2, angewendet auf die Herstellung von Cyclo(Pro-Tyr-D-Trp-Lys-Thr-Phe).

6. Ein Verfahren nach Anspruch 1 oder 2, angewendet auf die Herstellung von Cyclo(Pro-Phe-D-Trp-Lys-Thr-Phe).

7. Ein Verfahren nach Anspruch 1 oder 2, angewendet auf die Herstellung von Cyclo(N-Me-Ala-Tyr-D-Trp-Lys-Thr-Phe).